# EUROPÄISCHE PATENTSCHRIFT

(11) **EP 1 428 545 B1**
(45) Veröffentlichungstag und Bekanntmachung des Hinweises auf die Patenterteilung: **12.04.2006**
(21) Anmeldenummer: 02027767.9
(22) Anmeldetag: 11.12.2002
(51) Int. Cl.: A61M 25/00, A61M 25/06

(54) **Katheter**
Catheter
Cathéter

(43) Veröffentlichungstag der Anmeldung: 16.06.2004
(73) Patentinhaber: Abbott Laboratories Vascular Enterprises Limited, Dublin 2 (IE)
(72) Erfinder: Schneider, Ralph, 72414 Rangendingen (DE); Hartwig, Judith, 72359 Dotternhausen (DE); Nielsen, Stevan, 72108 Rottenburg (DE)
(74) Vertreter: Schmitz, Hans-Werner

(56) Entgegenhaltungen:
- EP-A- 0 792 655
- EP-A- 0 937 480
- WO-A-92/18193
- WO-A-96/16690
- DE-A- 3 907 549
- FR-A- 1 290 933
- US-A- 5 741 429
- US-B1- 6 387 075

## Beschreibung

Die Erfindung betrifft einen Katheter gemäß dem Oberbegriff des Anspruches 1.

Ein gattungsgemäßer Katheter, der beispielsweise aus dem US-Patent 5,569,200 bekannt ist, weist einen Katheterschaft auf, der üblicherweise als "Hypotube" bezeichnet wird.

Am proximalen Ende dieses Katheterschaftes ist ein Anschlussstück, vorzugsweise in Form eines Luer-Anschlussstückes, vorgesehen, das zum Anschluss einer Druckvorrichtung dient, über die durch im Anschlussstück vorgesehene Kanäle die Druckflüssigkeit zum Expandieren des Ballons am distalen Ende des Katheterschaftes eingeleitet wird.

Im Rahmen der Erfindung durchgeführte Untersuchungen haben gezeigt, dass die Übergangsstelle zwischen dem Hypotube und dem Anschlussstück sehr knickempfindlich ist. Hierbei kann der Benutzer den Katheterschaft abknicken, wenn er ihn aus der Verpackung und der Schutzhülle entnimmt oder wenn er die Druckvorrichtung anschließt. Die im Rahmen der Erfindung durchgeführten Untersuchungen haben hierzu ergeben, dass zumindest einer der Gründe für dieses unerwünschte Abknicken die Tatsache ist, dass der Katheterschaft einen sehr geringen Durchmesser aufweist, sodass der Benutzer den Katheterschaft beim Entnehmen aus der Verpackung oder beim Anschließen der Druckvorrichtung nicht ausreichend gut fühlt und ihn so unbeabsichtigter Weise abknicken kann. Das Abknicken ist deshalb besonders kritisch, da es zu einem Verschluss des Inflationslumens führen kann, der den Katheter funktionsunfähig macht.

Es ist daher Aufgabe der vorliegenden Erfindung, einen Katheter der im Oberbegriff des Anspruches 1 angegebenen Art zu schaffen, dessen Handlingeigenschaften verbessert sind und der insbesondere nicht knickanfällig ist.

Die Lösung dieser Aufgabe erfolgt durch die Merkmale des Anspruches 1.

Im gattungsgemäßen Stand der Technik gemäß dem US-Patent 5,569,200 ist die Knickempfindlichkeit zwar auch angesprochen und es wird das Vorsehen von Schlitzen im Katheterschaft als eine mögliche Lösung dieses Problems angegeben, jedoch sind diese Schlitze am distalen Ende vorgesehen, an welcher Stelle keine Kräfte quer zur Katheterlängsachse wirken, da nur Kräfte in Richtung der Katheterlängsachse aufgebracht werden. Insofern können diese Schlitze das eingangs erläuterte Problem nicht beseitigen, da beim gattungsgemäßen Katheter weiterhin die Gefahr des Abknickens bzw. sogar Abbrechens beim Aufbringen externer Kräfte, wie zuvor beschrieben, besteht.

Demgegenüber wird durch das bewusste Vorsehen eines Biegeabschnittes am proximalen Ende des Katheterschaftes erfindungsgemäß auf verblüffend einfache und nicht vorhersehbare Art und Weise das der Erfindung zugrundeliegende technische Problem gelöst, da das bewusste Vorsehen eines Biegeabschnittes gerade im kritischen Bereich des Katheterschaftes, der beim gattungsgemäßen Katheter besonders knickempfindlich ist, eine höhere Flexibilität schafft, die zwar ein gewolltes Abbiegen möglich macht, dadurch jedoch das unerwünschte Abknicken oder sogar Abbrechen sicher verhindert wird, da eine plastische Deformation des Katheterschaftes verhindert wird.

Aus der US 6,387,075 ist ein weiterer Katheter bekannt, der eine distal des gewebten Katheterschafts ins Hypotube eingeschnittene Spirale aufweist. Diese Spirale soll jedoch lediglich zur Verbesserung der Flexibilität des Katheterschafts im distal gelegenen Bereich des Katheters dienen. Die Spirale kann daher entsprechend der zuvor genannten US 5,569,200 die Gefahr des Abknickens des Katheters hinter dem Anschlussstück nicht vermeiden.

In der US 6,387,075 wird ferner beschrieben, dass zur Vermeidung des Abknickens des Katheters hinter dem Anschlussstück der proximale Katheterschaft auf einer Länge von etwa 6 bis 12 inch mit einem Polymer verstärkt oder durch eine Armierung stabilisiert wird. Mit dieser Verstärkung wird jedoch lediglich die Knickstelle in distale Richtung gegen das Ende der Verstärkung verschoben und darüberhinaus verringert die Polymer-Verstärkung die zur Verfügung stehende benutzbare Länge des Katheters und ergibt somit eine unerwünschte Verlängerung des Katheterschaftes.

Schließlich wird bei einer weiteren Ausführungsform zur Verstärkung des proximalen Katheterschaftes in der US 6,387,075 ein Kathether beschrieben, dessen proximaler Schaft statt aus einem Hypotube aus einem zwischen einem inneren und einem äußeren Polymer-Tube gelegenen Gewebe besteht. Diese Ausführungsform umgeht das Problem des Abknickens des Katheters durch den Einbau eines Katheterabschnittes, der nicht aus dem Hypotube, sondern aus neuen, zusätzlichen Schaft-Komponenten besteht. Hierbei ergeben sich jedoch große Nachteile durch die Verringerung der pushability and die aufwendige und teure Herstellung des Katheters.

Aus der EP 0 792 655 A2 ist ein weiterer Katheter bekannt, der einen Katheterschaft aufweist, dessen Profil elliptisch ausgebildet ist, was die Biegefestigkeit des Katheterschafts verbessern und damit seine Knickanfälligkeit vermindern soll.

Schließlich ist aus der WO 92/18193 A eine Venen-Verweil-Kanüle bekannt, deren Kanülenkörper im Bereich des Griffansatzes mit einer Flexur versehen ist. Diese Flexur soll Abwinkelungen über 90° ermöglichen, so dass beispielsweise ein Infusionsschlauch senkrecht zum Arm eines Patienten in der Venen-Verweil-Kanüle befestigt werden kann.

Da der Biegeabschnitt als eingeschnittene Spirale oder als eine Mehrzahl von versetzten Einschnitten mit geeigneten Dichtungen ausgebildet ist, ergibt sich der Vorteil einer sehr einfachen Produktionsmöglichkeit des Biegeabschnittes ohne Verwendung zusätzlicher Materialien, die auf zuverlässige Art und Weise eine Beschädigung des Katheterschaftes verhindert. Hierbei ergibt sich eine bewusste Materialschwächung, die zur gewünschten Erhöhung der Flexibilität und damit zum Knickschutz führt.

Die Unteransprüche haben vorteilhafte Weiterbildungen der Erfindung zu Inhalt.

Weitere Einzelheiten, Merkmale und Vorteile der Erfindung ergeben sich aus nachfolgender Beschreibung von Ausführungsbeispielen anhand der Zeichnung.

Es zeigt:
- Fig. 1: eine schematisch stark vereinfachte Darstellung eines erfindungsgemäßen Katheters,
- Fig. 2+3: eine der Fig. 1 entsprechende Darstellung möglicher Ausbildungen des Biegeabschnittes in Form versetzter Einschnitte,

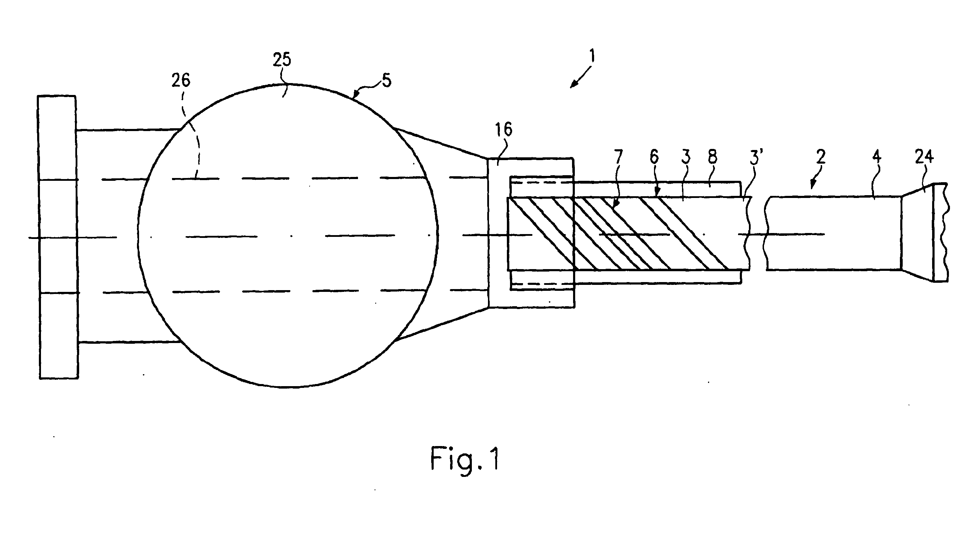

In Fig. 1 ist ein erfindungsgemäßer Katheter 1 dargestellt, der einen Katheterschaft 2 aufweist. Der Katheterschaft 2 ist mit einem proximalen Ende 3 und einem distalen Ende 4 versehen, die in Fig. 1 stark verkürzt dargestellt sind. Am distalen Ende 4 ist ein schematisch nur angedeuteter Ballon 24 befestigt. Ferner weist der Katheter 1 ein Anschlussstück 5 auf, das üblicherweise als Luer-Anschlussstück ausgebildet ist. Dieses Anschlussstück 5 ist am proximalen Ende 3 des Katheterschaftes 2 befestigt und kann ein üblich ausgebildetes Gehäuse 25 mit einem Durchtrittskanal 26 aufweisen, der mit dem Katheterschaft 2 in Strömungsverbindung steht. Dieses Anschlussstück 5 wird mit einer Druckvorrichtung (Inflationdevice) verbunden, um den Ballon 24 aufzuweiten.

Das proximale Ende 3 des Katheterschaftes 2 des erfindungsgemäßen Katheters 1 ist mit einem Biegeabschnitt 6 versehen, dessen Biegsamkeit größer ist, als diejenige des sich an das proximale Ende 3 anschließenden Abschnittes 3' des Katheterschaftes 2. Dieser Biegeabschnitt 6 verhindert ein ungewolltes Abknicken auf die eingangs erläuterte Art und Weise.

Wie in Fig. 1 dargestellt, ist der Biegeabschnitt 6 als eine in den Katheterschaft 7 eingeschnittene Spirale 7 mit einer wählbaren Anzahl von Spiralabschnitten ausgebildet.

Die Spirale 7 ist zur Verhinderung eines Flüssigkeitsaustrittes mit einer Dichtung 8 versehen, die als eine auf dem proximalen Abschnitt 3 befestigte, vorzugsweise aufgeklebte, Hülse oder Schlauch ausgebildet sein kann.

Wie Fig. 1 verdeutlicht, ist das proximale Ende 3 mit seinem Biegeabschnitt 7 und der darüber angeordneten Dichtung 8 mit einem Übergangsteil 16 des Anschlussstückes 5 verbunden, sodass der knickgefährdete Bereich des Katheterschaftes 2 gegen ein ungewolltes Abknicken geschützt ist.

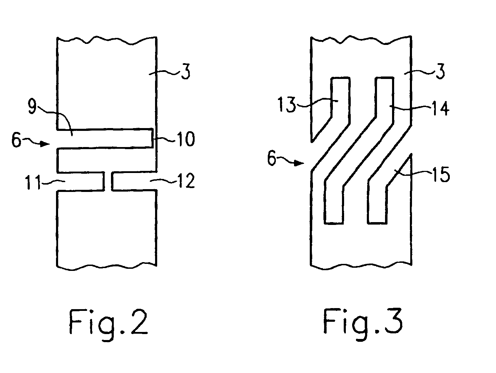

In den Fig. 2 und 3 sind alternative Ausführungsformen für den Biegeabschnitt 6 dargestellt. Bei der Ausführungsform gemäß Fig. 2 ist der Biegeabschnitt 6 am proximalen Ende 3 durch eine Mehrzahl von versetzten Schlitzen ausgebildet, die im Beispielsfalle durch zwei Schlitze 9 und 11 repräsentiert sind. Die Schlitze 9 und 11 reichen bis zu Stegen 10 bzw. 12, aus deren Anordnung sich die Versetzung ergibt. Die Schlitze können in ihren Dimensionen (Länge, Breite, Winkelstellung) an unterschiedliche Einsatzbedingungen angepasst werden.

In Fig. 3 ist der Biegeabschnitt 6 am proximalen Ende 3 ebenfalls durch eine Mehrzahl von versetzten Schlitzen 13, 14 und 15 gebildet, die bei dieser Ausführungsform nicht wie in Fig. 2 in Umfangsrichtung, sondern im Wesentlichen in Längsrichtung des proximalen Endes 3 verlaufen. Die Schlitze 13 und 15 weisen hierbei zwei Schlitzabschnitte auf, von denen einer in den Randbereich des proximalen Endes 3 verläuft, während der Schlitz 14 drei im Winkel zueinander angeordnete Schlitzabschnitte zwischen den Schlitzen 13 und 15 aufweist.

Auch Katheter, die keinen Ballon aufweisen, sind als erfindungsgemäße Katheter zu verstehen.

## Patentansprüche

1. Katheter (1)
- mit einem Katheterschaft (2), der ein proximales Ende (3) und ein distales Ende (4) aufweist, und
- mit einem Anschlussstück (5), das am proximalen Ende (3) des Katheterschaftes (2) angeordnet ist,
**dadurch gekennzeichnet,**
- **dass** der Katheterschaft (2) in seinem proximalen End abschitt mit einem Biegeabschnitt (6) versehen ist,
- **dass** der Biegeabschnitt (6) eine Biegsamkeit aufweist, die größer ist als diejenige des sich an den proximalen End abschuitt distal anschließenden Abschnitts (3') des Katheterschaftes (2), und
- **dass** der Biegeabschnitt (6) eine in (3) den Katheterschaft (2) eingeschnittene Spirale (7) aufweist, oder aus einer Mehrzahl in den Katheterschaft verstzt eingeschnittener Einschnitte (9, 11 bzw. 13 bis 15) gebildet ist.

2. Katheter nach Anspruch 1, **dadurch gekennzeichnet, dass** am distalen Ende (4) des Katheterschafts (2) ein Ballon (24) befestigt ist.

3. Katheter nach Anspruch 1 oder 2, **dadurch gekennzeichnet, dass** der Biegeabschnitt (6) mit einer Dichtung (8) versehen ist.

4. Katheter nach Anspruch 3, **dadurch gekennzeichnet, dass** die Dichtung (8) als am proximalen Ende (3) des Katheterschaftes (2) befestigte, vorzugsweise verklebte, Hülse oder Schlauch ausgebildet ist.

5. Katheter nach einem der Ansprüche 1 bis 4, **dadurch gekennzeichnet, dass** das Anschlussstück (5) als Lueranschlussstück ausgebildet ist.

## Claims

1. A catheter (1), comprising
- a catheter shaft (2) having a proximal end (3) and a distal end (4), and
- a fitting (5) arranged at the proximal end (3) of the catheter shaft (2)
**characterized in that**
- the catheter shaft (2) is provided at its proximal end portion with a bending section (6),
- the bending section (6) has a flexibility greater than that of the section (3') of the catheter shaft (2) arranged distal to the proximal end portion, and
- the bending section (6) has a spiral cut into the catheter shaft (2) or consists of a plurality of cuts (9, 11 and 13 to 15, respectively) cut offset into the catheter shaft.

2. The catheter of claim 1, **characterized in that** a balloon (24) is attached at the distal end (4) of the catheter shaft.

3. The catheter of claim 1 or 2, **characterized in that** the bending section (6) is provided with a seal (8).

4. The catheter of claim 3, **characterized in that** the seal (8) is designed as a sleeve or tube being fixed, preferably glued, to the proximal end (3) of the catheter shaft (2).

5. The catheter of one of claims 1 to 4, **characterized in that** the fitting (5) is designed as a luer fitting.

## Revendications

1. Cathéter (1)
- comportant une tige de cathéter (2), qui comporte une extrémité proximale (3) et une extrémité distale (4), et
- comportant une pièce de raccordement (5), qui est agencée sur l'extrémité proximale (3) de la tige de cathéter (2),
**caractérisé**
- **en ce que** la tige de cathéter (2) est munie d'un tronçon flexible (6) dans sa zone d'extrémité proximale,
- **en ce que** le tronçon flexible (6) a une flexibilité supérieure à celle du tronçon (3'), qui prolonge la zone d'extrémité distale, de la tige de cathéter (2), et
- **en ce que** le tronçon flexible (6) comporte une spirale (7) incisée dans la tige de cathéter (2) ou est formé par une pluralité d'entailles (9, 11 et 13 à 1 5) incisées dans la tige de cathéter (2).

2. Cathéter selon la revendication 1, **caractérisé en ce qu'**un ballonnet (24) est fixé sur l'extrémité distale (4) de la tige de cathéter (2).

3. Cathéter selon la revendication 1 ou 2, **caractérisé en ce que** le tronçon flexible (6) est muni d'une garniture d'étanchéité (8).

4. Cathéter selon la revendication 3, **caractérisé en ce que** la garniture d'étanchéité (8) est réalisée sous forme de manchon ou tuyau, fixé, de préférence collé, sur l'extrémité proximale (3) de la tige de cathéter (2).

5. Cathéter selon l'une quelconque des revendications 1 à 4, **caractérisé en ce que** la pièce de raccordement (5) est réalisée sous forme d'embout Luer.
